# EUROPEAN PATENT SPECIFICATION

(11) **EP 2 512 444 B1**
(45) Date of publication and mention of the grant of the patent: **22.03.2017**
(21) Application number: 09828425.0
(22) Date of filing: 17.12.2009
(51) Int. Cl.: A61K 9/02, A61K 41/00, A61K 35/64, A61K 36/17, A61K 36/185, A61K 36/24, A61K 36/35, A61K 36/42, A61K 36/53, A61K 36/71, A61K 36/736, A61K 36/81

(54) **MIXTURES OF PLANTS IN SPAGYRIC MOTHER TINCTURE FOR DRY COUGH IN BABIES**
PFLANZENGEMISCHE IN EINER SPAGYRISCHEN URTINKTUR GEGEN TROCKENEN HUSTEN VON BABYS
MELANGES DE PLANTES DANS UNE TEINTURE-MERE SPAGYRIQUE CONTRE LA TOUX SECHE CHEZ LES BEBES

(43) Date of publication of application: 24.10.2012
(73) Proprietor: Marzano Ancora, Monica, 6500 Bellinzona (CH)
(72) Inventor: Marzano Ancora, Monica, 6500 Bellinzona (CH)
(86) International application number: PCT/IB2009/007821
(87) International publication number: WO 2010/067199

(56) References cited:
- CN-A- 1 739 645
- US-B1- 6 541 045
- DATABASE WPI Week 200143 Thomson Scientific, London, GB; AN 2001-399065 XP002618347, & CN 1 290 538 A (WAN D) 11 April 2001 (2001-04-11)
- "Suppositorien, Vaginalzubereitungen, Stifte"; "Chapter 13" In: Bauer, Frömming, Führer (Eds): "Pharmazeutische Technologie", 1993, Georg Thieme Verlag, Stuttgart, XP002618348, ISBN: 3-13-692504-1 vol. 4, pages 272-277, pages 272-274, paragraph 1
- "Homöopatische Zubereitungen und Darreichungsformen"; "Chapter 19" In: Bauer, Frömming, Führer (Eds): "Pharmazeutische Technologie", 1993, Georg Thieme Verlag, Stuttgart, XP002618349, ISBN: 3-13-692504-1 vol. 4, pages 382-388, page 386, right-hand column, paragraph 7 page 387, right-hand column, paragraph 4-6
- J. Tukker: "Rectal and vaginal drug delivery"; "Chapter 34" In: M.E. Aulton (Ed): "Pharmaceutics. The science of dosage form design", 2002, Churchill Livingstone, Edinburgh, XP002618350, ISBN: 0443055173 vol. 2, pages 534-543, pages 537-538
- SCHELLERER S: "DEM SCHNUPFEN EIN SCHNIPPCHEN SCHLAGEN", PHARMAZEUTISCHE ZEITUNG, GOVI VERLAG, ESCHBORN, DE, vol. 148, no. 37, 11 September 2003 (2003-09-11), pages 16-25, XP001247323, ISSN: 0031-7136

## Description

### Subject and / or technical field

This invention is related to the production of suppositories made of a mixture of spagyric plants for the dry cough of children.

### Exposure of the invention:

### State of the art

In the market are commercially available different allopathic products that contain ingredients that have the ability to be cough suppressants such as decongestants, antihistaminic, antitussives and expectorants. Unfortunately these properties are associated with various side effects. In the sedation process of the cough are widely used ingredients based on opioids. Only few products and scarce information are available of ingredients that have a natural base for children.. Numerous antitussive formulations were reported in the literature. Eg, Kuger and others, U.S. Pat. No.3, 789.125, describes an antitussive containing the active ingredient alo-substitute of 2amino-benzilamine-amide. Stenger et al., U.S. Pat.No.3,958,002, describe an anti-tussive medicament which contains ortho-cresotamide derivatives.

There are few references of antitussive derivatives that have a natural origin. None of them are referred to be used in paediatric children or newborn.: Raiko begs C., Pat. USNo.5, 660.833, "antitussive composition". This invention incorporates plant extracts that suppress the effect of antitussive cough due to various aetiologies. Raiko C.Mendica, Pat. U.S .No. 5,660,833,"Antitussive composition": This invention is an antitussive composition that contains extracts of natural plant which are non-addictive and suppresses coughing due to various aetiologies. Panchapagesa Muthuswamy Murali, U.S. Pat.No.6,197,306, present an invention of an herbal composition (and the process the making) focussed for the treatment of chronic obstructive pulmonary disease, bronchitis and respiratory disorders. Doshi et. Al, U.S. Pat.No. 2006,0257507 A1. This invention relates to an herbal composition and a process for preparation of the homogenous cough syrup, which is non-alcoholic, non-sedating and non-freezing for the treatment of chronic respiratory disorders such as cold, cough, allergic asthma, seasonal allergic rhinitis, pharyngitis, laryngitis and the like. Alvin Forester Rigby, U.S. Pat.No.6,582,702 B2, With this invention he explains how to make an herbal composition based on extracts of different plants. The composition acts as a tonic to improve respiration, aid in the elimination of toxins and improves the overall vitality. CN 1 739 645 discloses a medicinal preparation comprising seven herbal medicinal materials among which Ephedrae and armeniacae amara for use in treating cough. The preparation can be in the form of a tablet, capsule, oral liquid, syrup, granular formulation, pill, powder, injection, suppository, emulsion, patch, etc.

Most of these invention emphasize the scarcity of information available regarding the combination of mixtures of plants even if those are in the common use in some countries.

The majority of the allopathic products that are in the market contain cough suppressant ingredients with one of the following properties: decongestants, antihistaminic, antitussives and expectorants. All of those properties are associated with various side effects.

Only few of them are for dry cough of babies from zero months and are of natural origin.

### Objective

This invention aims to develop a product that is safe for rectal administration as defined by the claims. It concerns suppositories made of a mixture of spagyric plants that are created following the dr. Zimpel method (Drosera, Ephedra, Hydrastis canadensis, Thymus vulgaris, Amigdala amara, Propolis, Catharanthus roseus, Bryonia alba, Sambucus nigra, Mandragora officinalis and variants of the herbal species) The product is meant to remove in preferences the dry cough in children from zero months onwards and that can be also used in other types of cough mixture.

With this invention we want to solve: the states of irritation due to dry cough; start/help the melting process of catarrh; avoid the almost asthmatic attacks of cough that have some babies; avoid the formation of attacks of whooping cough (type pertussoide) that in some cases lead vomit in babies, avoiding the annoying tickle due to the beginning of the formation of phlegm that is unable to detach by himself; to transform the states of cough mixture to a solving expectorant form.

### Solution

The aim is achieved thanks to the characteristics of claim 1.

### Advantages

The claims show beneficial developments. The invention has the following advantages: it has an antitussive activity on children in a natural way. The plants are processed in a way that eliminates the various alkaloids that may cause side effects, the way of administration allows the proper and safe absorption of active principle dissolved in the plant.

The use of the herbs and plants to treat diseases or cold is commonly used in most countries. At present time there are not enough data available to make natural mixtures for dry cough in children ranging from 0 months onwards.

For this invention we consider the following mixture of spagyric plants described further below that can be used for products with rectal administration. The use of each plant of the invention is documented. A summary of the individual use of each plant is described hereafter.

### Introduction

### Summary in the mechanism of cough

The cough mechanism is an explosive expiration which provides a mean for clearing the tracheal and bronchial trees of accumulated secretions and/or foreign bodies. Coughing is among the most frequently reported cardio-respiratory symptoms and perhaps the most common symptom for which medical attention is sought. The cough mechanism is initiated by an appropriate stimulus which elicits a deep inspiration. Coughing is caused by an extremely wide range of different factors. For instance, coughing is produced by inflammatory mechanisms, mechanical disorders chemical and thermal stimulation of the cough receptors.

Drosera rotundifolia: The Rosolida (Drosera rotundifolia L., 1753) is a carnivorous plant of the family of Droseraceae. It is a herbaceous plant of 10-20 cm high, with leaves obovate and with a long stalk, arranged in basal rosette, equipped with long tentacles with purple hairs that secrete a viscous liquid drops, with which it traps small insects. The tentacles fold on the prey after capture. It blooms from April to September with small white flowers. Has a cosmopolitan distribution, limited in the northern hemisphere. Prefers damp places and swamps, and alpine meadows with moist acid substrate, at altitudes from 300 to 1600 meter. This plant is found in both North America and Europe and Asia. *Clinical indications and main activity:* antitussive, broncospasmolitica, expectorant. The Drosera rotundifolia is used in herbal medicine for its properties and as a soothing expectorant cough. It is used in homeopathy for the following activities at different concentrations. *Respiratory indications:* larynx or tracheobronchitis with spasmodic fits of coughing, especially at night. At the larynx level there is catarrhal irritation of the mucous membrane with a sensation of tickling or of a foreign body which causes the stimulation of cough; at tracheal level the irritation causes a choking cough spasmodic, often at night, feeling of oppression of the chest as if the talking and coughing of the air was retained and could not be exhaled. In some cases the dry cough can lead to the sensation of agonizing, the fits of coughing come one after the other so violently that the subject can't take a breath and has the felling of a choke; this kind of cough is so violent that the patient keep his belly for breath and is sometimes followed by nausea, vomiting, and eventually by bleeding of the sinuses. The plant is also indicated in cough similar to pertussis, in vomiting triggered by accesses that cause congestion of the face. It is also proposed for the cure of osteoarticular problems: for example the inflammatory arthritis due to some form of viral rheumatism. Sensation of soreness or distortion in the joints, bone pain, especially of the long bones and vertebrae, joint pain and/or at the shoulder that can bruise and/or pierce and that can get worse on palpation. The remedy described is particularly useful in cases of "rheumatism of the hip" in the young child. The symptoms generally worsen at night, according to the lying, with the warmth of the bed, laughing, talking or singing. It gets better with movement and with the pressure of the hands. *Other suggested utilization:* Asthma (extrinsic with spasmodic cough and/or with the feeling of tightness in the chest), bronchitis (chronic peptic ulcer or gastritis), emoptisis (caused by the efforts of coughing), pharyngitis (acute stages), cold (subacute with ipofonia or laryngitis), tracheitis.

Cough (acute spasmodic and paroxysmal, laryngeal reflex and/or spasmodic cough in infant with measles or whooping cough and/or irritative and spasmodic cough) In the treatment of asthma it is often combined with: Grindel, Euphorbia, Seneghe. The used part is the entire flowering plant. *Main constituents:* naftoquinoni (Plumbagin, its methyl ether); flavonoids. Detail of its main constituents: 3-8-dihydroxy-leaf Plumbagin 100 ppm hydro ,7-methyl juglone leaf ,7-methyl juglone whole plant 250 ppm, 9 ppm in peltatone whole plant, Biramentaceone whole plants 14 ppm, 1.000 ppm Droserone leaf, Droserone-5-O -beta-d-glucoside aerial part, Gossipetina leaf Gossipin leaf, leaf gossipitrina Iso-500 ppm, Myricetin whole plant, leaf Plumbagin 5.000 ppm, Plumbagone whole plant, acidic polysaccharide (Drosera capensis) leaf, quercetin whole plant, Quercetol whole plant. *Pharmacology and pharmacodynamics:* The extract resulted antispasmodic against bronchospasm induced by acetylcholine and barium chloride, antitussiv and secretolitic (loosen phlegm). The Plumbagin is antimicrobial in vitro against Gram-(+), Gram-(-), influenza viruses, mushrooms and protozoa. In vitro is immunostimulant. The presence of Plumbagin and its antibacterial action explain the effectiveness of the plant. It is also useful in infections in other parts of the respiratory tract. *Contraindications:* Breastfeeding, pregnancy (unsupervised), inflammation of skin or mucous membranes (application of fresh plant).

Ephedra sinica: belonging to the family Ephedraceae . The most used is Ephedra sinica Stapf, but also other species are used: Ephedra distachya (European) trifurcata Ephedra (E. viridis), Ephedra nevadensis (North American), Ephedra American (North American), Ephedra gerardiana Wall. (India and Pakistan), nebrodensis Tineo Ephedra, Ephedra intermediate Schrenk et CA Mey, Ephedra equisetina Bge. The Ephedra is a shrub from 40 cm to 1 m tall. Stem bented, then ascending; branches glaucous green, opposite or sorted, consisting of two rigid segments of 2-4 cm. The dried young stems of the perennial plant are up to 30 cm long and have a diameter up to 2 mm, gray-green and slightly rough. From the nodes spaced 3-6 cm spreads short leaves. The cross section of stem shows 6-10 bundles of fibers. The parts that are normally used are the aerial parts (stems and branches). *Main constituents:* Protoalcaloidi (2.5-3%), including: I-ephedrine (0,75-1%), d-efedrinapseudoefedrina, norephedrine, N-methylephedrine, benziletilammina, tannins, saponins, flavonoids. *Clinical indications and main activity:* anorectic, antiallergic, antiasthma, broncospasmolitic, heartstimulant, decongestant, diaphoretic, hypertensive, sympathomimetic (alpha-and beta-adrenergic), stimulant (CNS). Probable additional activity to the mixture: decongestant and alpha and beta adrenergical. Secondary activities: anti-inflammatorical and diuretical. *Pharmacology and pharmacodynamics* This plant is famous especially for its content of ephedrine - extracted for the first time from Ephedra sinica - which has been used in biomedicine to treat bronchial asthma, although it is now very rarely used due to significant and undesirable hypertensive side effects. The plant contains many constituents which in combination in their natural state act synergistically and can be used in lower doses to produce the typical therapeutic effects (relaxation of bronchial muscle, increase the periphetic blood flow and increase the sweating and the thermogenesis) reducing the risk of side effects. The action of the plant depends on its content in proto-alkaloids, particularly I-and d-ephedrine and pseudoephedrine. The I-ephedrine is similar in effect to adrenaline, but is much less active, it is better absorbed by the gastrointestinal tract and has a longer duration. Compared to adrenaline, the ephedrine has a greater action in the CNS, similar (but less strong) to amphetamines. It exerts a significant alpha-adrenergic action (interaction with 1-adrenoceptor), tightening the peripheral vasculature and increasing blood pressure, lowering the tone and intestinal motility and increasing the tone of sphincters, relaxing the bronchial smooth muscle, dilating the pupils, increasing blood sugar levels and contracting the muscles of the reproductive system. This represents the first stage of sympathetic activation (adrenaline) and is potentially hypertensive. This first effect is, however, soon contrasted and eclipsed by the action of d-pseudoephedrine, which is strongly beta-adrenergic. Thus the perfusion of the periphery and skeletal muscle is increased, the bronchial airways are dilated and there is a relaxation of intestinal muscles and reproductive system. D-norpseudoephedrine is a CNS stimulant. The ephedrine and the pseudoephedrine are anti-inflammatory. One of the effects of ephedra alkaloids are the bronchodilation and this property is used to treat asthma. The effect reaches its peak in one hour and lasts up to 5 hours. Ephedra is also used as a nasal decongestant in the case of allergic rhinitis.

Hydrastis canadensis L.: It can be commonly found in the mountain of North America. It belongs to the family of Berberidacee or buttercups. The used parts are the rhizome and the roots. It is a perennial herb (Hydrastis canadensis) originating in North America, cultivated in Europe for its root from which are extracted a drug containing various active principle: *Its Main constituents:* iso-quinolic alkaloids: Idrastin, berberine, berberastin, canadin, traces of candalina and cannadalina, phytosterols, lipids, chlorogenic acid, essential oil, resin and carbohydrates, vitamins A, B and C, minerals (especially phosphorus); All these elements confer different properties: Antiseptic, astringent, haemostatic and anti-inflammatory. The alkaloids that are contained in the root have an anti-inflammatory and antiseptic activity. *Clinical indications and main activity:* In traditional medicine the root is a part of many preparations, it is used in particular to promote the health of the mucous membranes of the respiratory tract, gastrointestinal, urogenital, vaginitis. Mostly used by Native Americans and early settlers, the root of goldenseal is still used as a sedative for the inflammation of mucous membranes and as a natural remedy for colds and flu. For internal use is employed as a bitter stomachic and control of uterine bleeding. It can be used in liquid extract, tablets. To be used only for short periods of time and only at specific doses. It is a natural antibiotic, which is useful to normalize the functions of the mucous membranes. The berberine has also an antihistaminic action, antimicrobial activity for the staphylococcus, streptococcus, the Pseudomonas aeruginosa, Neisseria gonorrhea, meningitis, triconomas vaginalis, Salmonella. Extracts and Idrastin in particular is used to contain uterine bleeding and relieve menstruation pain. The goldenseal also has antibiotic action, immunostimulant, anticonvulsant, sedative, uterotonica, choleretic. The goldenseal is used with good results in the following cases: in the presence of nasal catarrh, throat and bronchial. Acts effectively by regenerating the cells of the mucous membranes and by doing so it decreases the secretion of mucus, reduces the congestion and inflammation that accompany the states of catarrh. It is administered internally and externally (gargle). It is used for copious menstruation and metrorrhagia (uterine bleeding) acting as a constrictor on the uterus. For external use it is used as: disinfectant, healer, for vaginitis and vaginal discharge (in the form of washing); periodontal disease and gingivitis (gum inflammation); Conjunctivitis (eye irritation): by washing(Antimicrobial activity). *Contraindications:* It is not recommended during pregnancy and to those suffering from hypertension and cardiovascular diseases. Its toxicity is related to the dosage, therefore is prescribed for a limited time, because it destroys intestinal flora. It can cause seizures, and is better not to use this plant for therapeutic purposes. Its alkaloids are potentially toxic and can have ulceration effects. It's prolonged use may decrease the absorption of the B group vitamins. It has interactions with heparin because it antagonizes the anticoagulant effect.

Thymus vulgaris: Lamiaceae family. The thymus originates in the western Mediterranean region. It grows naturally throughout the Mediterranean area up to 1500 m. It prefers limestone and well drained soils. It grows in sunny places and doesn't tolerate wet and cold winter. Thymus is a plant that has its bloom from May to October. It has very dense branches and leaves, small shrub and is particularly bushy. It is cultivated in France, Spain, Greece, Portugal and the United States. *Other species*: the source of the essential oil of thyme is not only the Thymus vulgaris but also can be used the Thymus zygis L. and Thymus pulegioides L. The essential oil of thyme is derived from Moroccan Thymus saturoides, that is rich in borneol. *Major constituents* Essential oil, which varies in quality: thymol (45-48% red, 0% sweet), carvacrol (2.5-3.5% red, 0.7% sweet), 1,8-cineole (3.6-15.3% red, 0% sweet), geraniol (0% red, 30.4% sweet), geranyl acetate (0% red, 50.1% sweet), beta-caryophyllene (1.3-7.8% red, 4.1% sweet), alpha-pinene (0.5-5.7% red, 0 sweet%), p-cymene (18.5-21.4% red, 0% sweet), terpineolene (1.8-5.5% red, 0% sweet), borneol, linalool, bornyl acetate and Linalyl, thymol methyl ether. Flavonoids: apigenin, luteolin, timonina, naringenin and others. *Clinical indications and main activity:* antibacterial, antifungal, antimicrobial, antioxidant and antispasmodic, carminative, expectorant (stimulant), rubbing. Other: it contains caffeic acid, tannins. Asthma, bronchitis (dry), candidiasis, catarrh, gastric colic, convalescence, diarrhea, dysmenorrhea, dyspepsia, flatulence, infection (respiratory tract), infection (respiratory tract with dry catarrh), infection (gastrointestinal), inflammation (respiratory tract), skin fungus, intestinal parasites, whooping cough, tonsillitis (locally), cough. Secondary Activities: anthelmintic, astringent. *Toxicity* It hasn't toxicity under specific doses. There is no proved increase in the frequency of malformations or other adverse effects on the fetus despite the consumption by a large number of women. There is also a lack of data from animal studies. It is compatible with breastfeeding. Occasional allergic reactions. *Pharmacology and pharmacokinetics* A study on the metabolism of thymol and carvacrol in animal models indicate that the urinary excretion of metabolites is fast. Although a certain amount of both compounds was excreted without being metabolized, it was detected a high level of oxidative metabolite. *Pharmacodynamics Antispasmodic* Thymol, carvacrol and essential oil as a whole demonstrated a relaxing effect on the trachea and on the ileum (inhibit the phasic contractions of the ileum). But the essential oil and its components are not solely responsible for the antispasmodic activity of Thymus; Indeed, the dried extracts of thymus with very low levels of thymol and carvacrol showed an antispasmodic activity in vitro. It is probable that the flavonoids are partly responsible for this activity, by blocking the influx of ions Ca. *Antibacterial* The essential oil is without doubt the most important part for this activity. Thymol (more effective) and carvacrol (less effective) are strong antibacterial and act on the perforation of the cell membrane. It is demonstrated a synergic action between carvacrol and thymol. The essential oil is extremely active on bacteria. It inhibits or kills E. coli, Lyster monocytogenes and many other bacteria (1 part in 800). The essential oil (1 part in 500) inhibits Clostridium botulinum. Even the fumes of the essential oil are strong antibacterial. *Antifungal* Thymol and carvacrol and the essential oil of Thymus (CT unspecified) have good fungistatic and fungitossic spectrum (Cryptococcus neoformans, Penicillium spp, Aspergillus flavus). The essential oil is very effective against eight different strains of dermatophytes and against various fungi (Rhizoctonia solani, Pythium ultimum var. Ultimum, Fusarium solani, Colletotrichum lindemuthianum). *Antioxidant* The methanol extract of thyme has strong scavenging activity of hydroxy radicals (in vitro). Both as volatile fraction and as flavonoid have shown to exert antioxidant action. Thymol, carvacrol and the para-cymene-2 ,3-diol showed excellent antioxidant activity in vitro (the para-cymene-2 ,3-diol is the strongest). Rosemary acid inhibits lipoperossidation, decreases the production of superoxide and inhibits the oxidative effects of exogenous compounds. *Antiallergic and antiinflammatory* In vitro the Rosemary acid inhibits the classical complement path and thus the immunoemolysis of the erythrocytes. In animal models the rosemary acid inhibited passive cutaneous anaphylaxis. Thymol inhibits neutrophilic chemotaxis in vitro. Thyme oil inhibits in vivo the synthesis of prostaglandins.

Propolis. It is a resinous substance that bees collect from the buds and bark of plants. This is therefore a drug of purely vegetable origin even if the bees, after harvest, process with the addition of wax, pollen and enzymes produced by the bees themselves. The colour can vary greatly in shades of yellow, red, brown and black. The smell is highly aromatic. There are several theories about the origin of propolis. The currently most accepted is the one formulated by Rosch. He noted that bees collect resins from trees with their mandibles and then process them with their front legs, middle and back up to lead them into the bag pollen of the latter pair of legs. The theory that assumes the internal source of propolis into the hive is not credited as it has not yet been demonstrated. *Clinical indications and main activity* Propolis has antibiotic properties (bacteriostatic and bactericidal), anti-inflammatory, antifungal, antioxidant, antiviral, anesthetic, healing, antiseptic, immunostimulants, vasoprotective, anti-cancer. The most important property is to have all the properties above concentrated together in a single product of natural origin. *Major constituents* It is impossible to define a universally valid and exact composition of propolis as it is highly variable depending on the vegetation of origin, season and many other factors. From several studies from various areas on propolis there have been identified over 150 different biochemical compounds and others are being discovered even today. In order to simplify the main components they can be divided into five main groups: resins (45-55%), wax and fatty acids (25-35%), essential oils and volatile substances (10%), pollen (5%), organic compounds and minerals (5%). Going into detail between the components of interest they include: minerals (Mg, Ca, I, K, Na, Cu, Zn, Mn and Fe). Vitamins: B1 (thyamin), B2 (riboflavin), B6 (pyridoxine) C (ascorbic acid) and E (tocopherol). Enzymes: succinate dehydrogenase, glucose 6-phosphatase, acid phosphatase. Acids: feniletylester caffeic acid (CAPE) found in resins and organic compounds, phenol, adenosine triphosphate (ATP). Derivatives of benzoic acid: gentisic acid, salicylic acid, protocatechico, acid-3-HYDROXYBENZOATE, acid-4-hydroxybenzoate, gallic acid, acid-4-methoxybenzoic acid. Derivatives of cinnamic acid: caffeic acid, ferulic acid, isoferulic acid idrocaffeic acid, p-coumaric acid, o-coumaric acid, m-coumaric. Coumarins: coumarin, Esculetin, scopoletin. Alcohols: benzyl alcohol, Cinnamyl Alcohol, Phenylethyl alcohol, alcohol pentenyl, alcohol 3.5-dimedossibenzilico. Aldehydes: vanillin, isovanillin, aldehyde cinnamic. Flavonoids: flavones: 5-hydroxy-7, 4'-dimetossiflavone, acacetin, apigenin-methylether 7.4 ', crisin, pectolinarigenin, tettocrisin; flavonols: 3,5-dihydroxy-7, 4'-dimetossiflavone, betuletol, ermanin, galangin, isalpinin, isoramnetin, kaempferide, kaempferol, quercetin-3, 3'-dimethyl ether, quercetin , ramnazin, ramnetin, ramnocitrin; Flavanone: 5-hydroxy-7, 4-dimetossiflavanone, Isosakuranetin, Pinocembrin, pinostrobin, sakuranetin; dihydroflavonol: pinobaksin, pinobanksin-3-acetate. Terpenes are contained in resins and essential oils and give the characteristic odor to propolis; Hydrocarbons: caryophyllene, *α-*Guaiene, *β*-selinene. Alcohols sesquiterpenes: *β*-eudesmolo, guaiolo. Amminoacids, fatty acids, ketones, sterols, polysaccharides, lactones. Special mention deserves the group of flavonoids that are contained in large quantities in propolis (up to 20% by weight). The bees modify the structure of flavonoids, originally present in plants, removing the sugars contained in the organic compound by enzymes produced by their salivary glands. Flavonoids have properties of inhibiting enzymes that normally remove the protein coat of viruses. Similarly they can block the process of allergic reaction by preventing the release of substances (histamine and serotonin) from the cells, a phenomenon that occurs in the presence of allergens. Flavonoids block the production of prostaglandins causing the aging process.

Sambucus nigra L. *Family of Adoxaceae* It is a shrub or small tree 2 to 5 m tall. His has stem with bark gray-brown, warty, with large cracks that show the smooth white inner surface. His flowers appear in May and are small, creamy white with yellow anthers. The flowers are hermaphrodite and give small and shiny berries. The Sambucus, in the form of small tree or shrub, is found naturally in humid forests and is common in most of Europe. It lives in almost all temperate regions, up to 1000-1200 m. It is often cultivated for its flowers and fruit. The flowers are harvested from May to June. The berries are harvested in early autumn. The part used is represented by: leaves, flowers and berries. *Major constituents* The main constituents depends on the part of the plant. Flowers: flavonoids (up to 3% as rutin, quercetin and kampferolo), phenolic acids (eg chlorogenic acid), triterpenes (ursolic acid, acid 30-beta-idrossiursolico, oleanolic acid, alpha-and beta-amirin), oils, fats (linoleic acid, linolenic acid and palmitic, alkanes), and sterols. Leaves: triterpenes (see above), cyanogenic glycosides (ex.sambunigrina.), flavonoids (rutin and quercetin) and various fatty acids, alkanes, tannins. *Clinical indications and main activity:* Anti-catarrh, anti-inflammatory, diaphoretic It is used in: bronchitis, for catarrh (chronic nasal), fever, influenza, colds, in allergic rhinitis, in sinusitis (acute and chronic). Secondary activity Antiallergic. *Pharmacology and pharmacodynamics* There are few researches on this plant. Even the mechanism of its diaphoretic effect is poorly understood. Some recent studies seem to confirm the anti-inflammatory action is probably due to ursolic acid.

The berries contain significant levels of anthocyanins and are strongly anti-oxidants. Some compounds (sambuculina A palmitate, alpha-and beta-amirina) isolated from a closely related species (Sambucus formosana) are antihepatotoxic and can be used against the damage induced by carbon tetrachloride. Some excerpts appear to have a hypoglycaemic action. It is used as antirheumatic and in arthritis, rheumatic disorders as it promotes the removal of waste metabolites via urine and sweat. The berries are rich in vitamin C and have been traditionally used for rheumatism and erysipelas. It is also slightly laxative, and can be used for light diarrhea. For the respiratory disorder the flowers are a perfect home treatment for coughs, colds and flu. They also has a role in reducing the fever. They are a good diaphoretic that increases the elimination through the skin and balances the circulation. The flowers compels the tone the mucous membranes of the nose and throat and increase the resistance to infections, irritations and allergies. The infusion is relaxing and calming and causes slight sweating. It is commonly prescribed for problems of chronic catarrh and allergies. The brew is usually taken for 3-4 months before the season of allergic rhinitis and it may help to reduce the severity of the attacks, although it is rarely sufficient to solve the problem. Combined with Echinacea is a useful remedy for ear infections in children and adults. As indicated by research, the berries are an excellent small decoction for colds and respiratory infections. Possible combinations in fevers and flu-like infections: Eupatorium perfoliatum, Mentha piperita and Achillea millefolium. *Toxicity* There are no proof of the increase of the frequency of malformations or other adverse effects on the fetus despite the consumption by a large number of women. There is a lack of data from animal studies. It is compatible with breastfeeding.

Amygdala amara or bitter almond from the family of Rosacae Bitter almond oil is obtained from the pressing of the seeds and is a clear liquid when freshly prepared, with a characteristic aromatic odour and bitter taste. The oil of bitter almonds is used in pharmaceutical preparations because it is rich in hydrogen cyanide. The poisonous action of this extract was known since antiquity, but only in 1803 it was made official by Gehlen. The drug is made from the seeds of Prunus Amygadalus Stockes -bitter varieties- (Fam. Rosacae), the tree is native to western Asia and grown mainly in southern Italy and Sicily. The number of seeds is one, and is contained in an ovoid drupe slightly flattered. The almonds are oblong-ovate, with wrinkled skin; kept in boiling water it easily lose the skin. The conservation of bitter almonds has some problems, as it is necessary to prevent rancidity and aggression from insects. *Major constituents* The active principle are: Benzoic aldehyde, hydrogen cyanide (2-5%), Fatty oil 50%, (Oleum amygdalarum), protein, calcium oxalate, zinc, copper, amino acids, vitamins, amygdalin or amigdaloside (2-4%) cinaogenetic glucoside. Bitter almonds is distilled in water. It has a strong odour. This is achieved in water by cold pressure and letting it soak for 12-24 hours, then the alcohol is added and distilled in steam: the obtained water contains hydrogen cyanide (small part free and partly in the form of cyanide benzoic acid) and Benzoic aldehyde. *Pharmacology and pharmacodynamics* The amygdala is considered a bitter sedative of the breath center, a sedative of the cough center. The hydrocyanic acid is a powerful poison with elective action on the respiratory center. *Clinical indications and main activity:* cough of any kind, whooping cough, asthma, laryngospasm, spasmodic coughs. *Toxicology* hydrocyanic acid is the poison that causes a rapidly death by paralysis of the bulbar centers and the breath cente. Accidental poisoning can occur by the introduction of foods that contain such acid as the seeds of cherries, peaches and lunate bean (Phaseolus lunatus L.), The symptoms of poisoning is expressed through seizures made due to asphyxia, unconsciousness and death.

Bryonia alba : the Bryonia is a plant of the family of Cucurbits; *Major constituents* brionina, brioretina, briogenina, idrobrioretina, brionidina, brioresine, volatile oils, gum, tannins, fitosterina, sugars, enzyme, resin. *Clinical indications and main activity:* Bryonia root is used as a malingerer, emetic, and diuretic, also in combinations for various diseases of the gastroinestinal tract, respiratory tract, for all forms of arthritis, for metabolic disorders, for liver diseases and as prophylaxis in the therapy of acute and chronic infections. The emetic and laxative effectiveness is undisputed. The effectiveness of other application aren't verified. In homeopathy is used in treating diseases that manifest themselves slowly and is able to act positively on the state of mind. To prepare the remedy it is minced and then turned into pulp. The Bryonia is indicated in the treatment of febrile illnesses that develop slowly, such as influenza and acute illness. Bryonia is useful for rheumatic disorders, headaches, backaches, muscle and joint pain. *Toxicity* The following effects have been observed after ingestion of Bryonia root preparations: dizziness, vomiting, severe colic, severe sometimes watery and bloody diarrhea, kidney damage, abortion, nervous excitement, and convulsions. Bryonia contains cucurbitacins, some of which have strong cytotoxic properties.

Catharantus Roseus: It belongs to the Apocynaceae family. The leaves are oval-oblong. It is an evergreen plant about one meter tall. *Clinical indications and main activity:* It is used in many diseases like Hoodgking syndrome, diabetes and malaria. It has an antidiabetic action: it is said to have a efficacy similar to insulin, but it is toxic in large doses. The effect is also similar to digitalis and also has mild laxative action. *Major constituents* The active principle consist of: vinblastine and vincristine - that are used for anti-cancer activity (particularly for leukemia)-, reserpine, ibogaine, yohimbine, Raubasine. It has an alkaloid that is toxic for the heart. With oral use it may be hallucinogenic.

Mandragora officinalis belongs to the Solanaceae family. The part that is mostly used is the roots and the leaves. *Major constituents* The main components consist of tropanici alkaloids (atropine, scopolamine) which are present mainly in the root, mandragorina (isomer of iosciamina), atropine, scopolamine. It is a perennial plant. Officinarum Alraune is an hermaphrodite plant (has both male and female organs). Mandragora has a long history of medicinal use, though superstition has played a large part in its use. It contains Hyoscine which is the standard pre-operative medication given to soothe patients which reduces the bronchial secretions. It is also used to treat travel sickness. *Clinical indications* Main activity: tonic, cholagogue, stimulant, sedative, narcotic. *Pharmacology and pharmacodynamics* The fresh or dried root contains highly poisonous alkaloids which is cathartic, strongly emetic, hallucinogenic and narcotic. In enough quantities it induces a state of oblivion. It was very used in the past for its anodyne and soporific properties. The juice from the finely grated root was used to be applied externally to relieve rheumatic pains, ulcers and scrofulous cancer. It was also used internally to treat melancholy, convulsions and mania. When taken internally in large doses, however, it is said to excite delirium and madness. The root should be used with caution, and only under the supervision of a qualified practitioner.

### Brief on excipients for suppositories

The excipients are supposed to give the necessary texture and chemical and physical characteristics to the suppository. Their have a determining influence on the transfer and absorption of the drug. The excipients that are more commonly used can be grouped in 3 types: lipophilic excipients, water soluble excipients and idrodispellent excipients (with and without surfactants). The lipophilic excipients must melt at body temperature to make the drug contained in them available, but it must be sufficiently firm until few degrees before this temperature. The most common lipophilic excipients are cocoa butter and solid semisynthetic glycerides (both characterized by having good local tolerability). In our preparation we preferred to use solid semisynthetic glycerides for 2 reasons: 1) good tolerability of children 2) ease of working at very low temperatures without the affection of the active drug mixture 3) longer shelf life. In general, solid semisynthetic glycerides are mixtures of mono-and tri-glycerides of saturated fatty acids of 10 to 18 carbon atoms. It aggregate in white crumbly masses that feels greasy when touched.

**Some features are listed below (according to Italian Official Pharmacopoeia IX edition)**

| Index | Quantity |
|---|---|
| Melting point | 33 ° -36 ° C |
| Acid value | not exceeding 0.5 |
| Iodine value | not exceeding 3 |
| Saponification value | 225-245 |
| Substances saponified | No more than 0.5% |
| Index of peroxides | not exceeding 6 |
| Hydroxyl value | not exceeding 50 |

For the virtual absence of acids with double bonds (iodine value <3) semisynthetic glycerides have remarkable stability and the presence of mono-and di-glycerides gives them the capacity to incorporate water. If water quantity is very high, it may form emulsions A/O that would release the active principle slowly. The characteristics of solidification and hydrophilicity, the semisynthetic glycerides, can be adapted to various scope by changing the ratio "triglyceride / partial glycerides" and fatty acids that are suitable for the esterification of glycerol. In the market there are different products with characteristics that are similar to the semi-synthetic glycerides as the Witepsol H®15. Another category of products that are made of a mixtures similar to Witepsol H®15 are those contains hydrogenated vegetable fat, oil, cetyl alcohol and myristic, ect. We considered Witepsol H®15 the reference carrier for the solubilization, dispersion and stabilization of our blend of spagyric plants.

Spagyric method in German Pharmacopoeia :The methods 25 and 26 are used for production of spagyric mother tinctures. Zimpel are defined in the German Homeopathic Pharmacopeia (HAB);

### According to HAB-method 26 (from dried plant parts):

1 part of diminished drug (8000) is mixed with 3 parts purified water and 0,01 parts yeast (Saccharomyces cerevisiae). After finishing of the fermentation -with daily mixing at a temperatures between 20 and 25°C-, the mixture is distilled in water steam. At the side of the distillate for 1 part of drug 2 parts of ethanol 86%(m/m) is placed beforehand; the distillation ends when is obtained 10 parts of distillate mixture - distillate and ethanol (submitted beforehand)- for 1 part of drug. The residue of the distillation is pressed, dried and calcinated at about 400°C. The residue of the calcination (the ash) is then added to the distillate; after 48 hours the mixture is filtrated. This is the mother tincture (Urtinktur), which is identical with the 1. decimal dilution (D1). The 2. decimal dilution (D2) is obtained by: 1 part of mother tincture (=D1) and 9 parts of a mixture made of 2 parts of ethanol 30%(m/m) and 1 part purified water. Analogous is the production of further dilutions.

### According to HAB-method number 25 (fresh plant parts):

For HAB-method 25 (from fresh plant parts) the procedure is similar to HAB-method 26, There are the following differences: Fermentation: a relation of 1 part of fine crumbled plant with 1 part of purified water and 0,005 parts of yeast. Distillation: for 1 part of drug, 0,4 parts of ethanol 86%(m/m) are placed beforehand. The distillation ends as soon as there are 2 parts of a mixture made of distillate and the ethanol beforehand submitted, for 1 part of drug. The 1. decimal dilution (D1) is obtained by: 2 parts of mother tincture and 8 parts of a mixture made of 2 parts Ethanol 30%(m/m) and 1 part of purified water. The 2. decimal dilution (D2) is made by: 1 part of mother tincture (D1) and 9 parts of a mixture composed by 2 parts of ethanol 30%(m/m) and 1 part of purified water. Analogous is the production of further dilutions.

| Plant | Parts of plant initially used (B) | Parts of plant used at the beginning of dr. Zimmpel's methods (-A-) | Parts obtained after the process | Analysis made to control the "good" quality of the plant (-C-) |
|---|---|---|---|---|
| Drosera | Plant | Plant | Tincture | Macroscopy, Microscopy, Foreign matter, Total ash |
| Ephedra | Plant | Plant | Tincture | Macroscopy, Microscopy, TLC, Foreign matter, Loss on drying, Total ash, Assay |
| Hydrastis canadensis | Rhizome | Rhizome | Tincture | Macroscopy, Microscopy, TLC, Foreign matter, Loss on drying, Total ash, Ash insoluble in |
| | | | | HCl, Assay |
| Thymus vulgaris | Plant | Plant | Tincture | Macroscopy, Microscopy, TLC, Foreign matter, Water, Total ash, Ash insoluble in HCl, Assay |
| Amygdala amara | Almond | Almond | Tincture | Macroscopy, Microscopy, Loss on drying, Total ash |
| Propolis | Resin | Resin | Tincture | Macroscopy, TLC, Melting point, Total ash I |
| Catharanthus roseus | Whole plant | Whole plant | Tincture | Macroscopy, Microscopy, HPLC, Foreign matter, Loss on drying, Total ash |
| Bryonia alba | Root | Root | Tincture | Macroscopy, Microscopy, TLC, chemical test, Loss on drying, Total ash |
| Sambucus nigra | Flower | Flower | Tincture | Macroscopy, Microscopy, TLC, Foreign matter, Loss on drying, Total ash, Assay |
| Mandragora officinalis | Root | Root | Tincture | Macroscopy, Microscopy, TLC, 2 chemical tests Foreign matter, Total ash |

For all the plants above addition controls are made to check the microbiological limits, heavy metals, pesticides and mycotoxines. for all plants that contain alkaloids tests are made to prove their absence. A simplified method n° 25, dr. Zimpel 10 kg of plant and 10 kg of distilled water and 0.05 kg of yeast is mixed together. The mixture is left in fermentation for 3 weeks at a temperature of about 20-25°C To this mixture (20kg), 80 kg of distilled water is added. The whole is then distilled. The result has a approximated alcohol of 6%. 0.4% of ethanol is added to the 86% of the mixture. The whole alcoholic concentration is lowered to 20% (v/v). The residual of the distillate is placed in a stove and is heated to 400°C until it becomes aches. The ashes and the distillate is mixed together and then filtrated. On all mother tincture with potential toxicological effects from alkaloids a control of their total absence is performed (in accordance with the European Pharmacopoeia test 2.3.1).

Example of the certificate of analysis of each plant

| Product | **Drosera** | Test code: EDRO.06 |
|---|---|---|
| PROPERTIES | | |
| appearance | Edro.06 | colorless |
| Odor | Edro 06 | Typical oily-fruity, sour |
| Taste | " | Typical salty, slightly sour-oily, spicy |
| TEST OF IDENTITY | | |
| Thin-film chromatography (TLC) | " | Complies |
| UV - Spectroscopy | " | " |
| TEST OF PURITY | | |
| pH - value | 4.5-10.5 | 6.8 |
| Ethanol content in% (V / V) | 17-30 | 21 |
| Relative density d ²⁰₂₀ | 0.970-0.980 | 0.974 |
| Dry residue in% (m / m) | 0.05-0.75 | 0.10 |
| Microbiology in KBE/ml | GKZ<1 | Complies |
| Methanol content in %(V/V) | ≤0.1 | 0.04 |
| 2-Propanol content in %(V/V) | ≤0.05 | <0.01 |
| | | |
| Assessment: | In the points examined above the tested sample corresponds to the set requirements | |
| Comments | None | |

| Product | **Catharanthus roseus** | Test code: ECATH.02 |
|---|---|---|
| PROPERTIES | | |
| appearance | ECATH .02 | colorless |
| Odor | ECATH .02 | Dull woody - herbaceous - earthy |
| Taste | " | Bitter, spicy, earthy-spicy |
| TEST OF IDENTITY | | |
| Thin-film chromatography (TLC) | " | Complies |
| UV - Spectroscopy | " | Complies |
| TEST OF PURITY | | |
| pH - value | 7.5-13.00 | 10.2 |
| Ethanol content in% (V / V) | 16-30 | 20 |
| Relative density d ²⁰₂₀ | 0.970-0.980 | 0.973 |
| Dry residue in% (m / m) | 0.05-0.80 | 0.36 |
| Microbiology in KBE/ml | GKZ<1 | Complies |
| Methanol content in %(V/V) | | |
| 2-Propanol content in %(V/V) | | |
| | | |
| Assessment: | In the points examined above the tested sample corresponds to | |
| | the set requirements | |
| Comments | An additional test for the presence of alkaloids using GC-MS went negative, ie there were no detectable alkaloids | |

| Product | **Mandragora officinalis** | Test code: EMAN.02 |
|---|---|---|
| PROPERTIES | | |
| appearance | EMAN.02 | colorless |
| Odor | EMAN.02 | TYPICAL ALRAUNE - almond |
| Taste | " | TYPICAL ALRAUNE - almond |
| TEST OF IDENTITY | | |
| Thin-film chromatography (TLC) | " | Complies |
| UV- spectroscopy | " | Complies |
| TEST OF PURITY | | |
| pH - value | 8.2-10.8 | 10.4 |
| Ethanol content in% (V / V) | 17-22 | 21 |
| Relative density d ²⁰₂₀ | 0.973-0.977 | 0.975 |
| Dry residue in% (m / m) | 0.08-0.40 | 0.26 |
| Microbiology in KBE/ml | GKZ<1 | Complies |
| Methanol content in %(V/V) | ≤0.1 | 0.01 |
| 2-Propanol content in %(V/V) | ≤0.05 | <0.01 |
| | | |
| Assessment: | In the points examined above the tested sample corresponds to the set requirements | |
| Comments | The test for alkaloids pursuant SOP.QK.034 (analogous Ph.Eur., EXAM 2.3.1) with 10 ml evaporated to dryness Essence went negative, ie there were no alkaloids | |

| Product | **Hydrastis canadensis** | Test code: EHYD.04 |
|---|---|---|
| PROPERTIES | | |
| appearance | EHYD.04 | Colorless |
| Odor | EHYD.04 | Species-specific dull, woody-earthy |

| Taste | " | Conspecific Rauth - bitter-earthy |
|---|---|---|
| TEST OF IDENTITY | | |
| Thin-film chromatography (TLC) | " | Complies |
| UV- spectroscopy | " | Complies |
| TEST OF PURITY | | |
| pH - value | 7.3-11.9 | 8.8 |
| Ethanol content in% (V / V) | 17-30 | 21 |
| Relative density d ²⁰₂₀ | 0.970-0.980 | 0.975 |
| Dry residue in% (m / m) | 0.05-0.85 | 0.22 |
| Microbiology in KBE/ml | GKZ<1 | Complies |
| Methanol content in %(V/V) | ≤0.1 | 0.02 |
| 2-Propanol content in %(V/V) | ≤0.05 | <0.01 |
| | | |
| Assessment: | In the points examined above the tested sample corresponds to the set requirements | |
| Comments | The test for alkaloids pursuant SOP.QK.034 (analogous Ph.Eur., EXAM 2.3.1) with 10 ml evaporated to dry essence went negative, ie there were no alkaloids | |

| Product | **Sambucus nigra** | Test code: ESAM 26.08 |
|---|---|---|
| PROPERTIES | | |
| appearance | ESAM 26.08 | A little green-yellow-brownish |
| Odor | ESAM 26.08 | Aromatic, sweetish |
| Taste | " | Dull spicy-herbaceous, bitter, spicy |
| TEST OF IDENTITY | | |
| Thin-film chromatography (TLC) | " | Complies |
| UV- spectroscopy | " | Complies |
| TEST OF PURITY | | |
| pH - value | 7.5-13.00 | 11.5 |
| Ethanol content in% (V / V) | 16-30 | 19 |
| Relative density d ²⁰₂₀ | 0.970-0.980 | 0.977 |
| Dry residue in% (m / m) | 0.15-1.20 | 0.53 |
| Microbiology in KBE/ml | GKZ<1 | Complies |
| Methanol content in %(V/V) | ≤0.1 | 0.1 |
| 2-Propanol content in %(V/V) | ≤0.05 | <0.01 |
| Assessment: | In the points examined above the tested sample corresponds to the set requirements | |
| Comments | None | |

| Product | **Tymus vulgaris** | Test code: ETHY.05 |
|---|---|---|
| PROPERTIES | | |
| Appearance | ETHY.05 | Reddish brown-yellow-green |
| Odor | ETHY.05 | Typically aromatic and spicy |
| Taste | " | hot and spicy, thyme |
| TEST OF IDENTITY | | |
| Thin-film chromatography (TLC) | " | Complies |
| UV- spectroscopy | " | Complies |
| TEST OF PURITY | | |
| pH - value | 7.0-12.00 | 10.4 |
| Ethanol content in% (V / V) | 16-30 | 19 |
| Relative density d ²⁰₂₀ | 0.970-0.980 | 0.977 |
| Dry residue in% (m / m) | 0.05-0.85 | 0.56 |
| Microbiology in KBE/ml | GKZ<1 | Complies |
| Methanol content in %(V/V) | ≤0.1 | 0.07 |
| 2-Propanol content in %(V/V) | ≤0.05 | <0.01 |
| | | |
| Assessment: | In the points examined above the tested sample corresponds to the set requirements | |
| Comments | None | |

| Product | **Ephedra** | Test code: EEPH 26.01 |
|---|---|---|
| PROPERTIES | | |
| Appearance | EEPH 26.01 | Pale yellow-green |
| Odor | EEPH 26.01 | Aromatic, sweet |
| Taste | EEPH 26.01 | Intense spicy-bitter, slightly pungent |
| TEST OF IDENTITY | | |
| Thin-film chromatography (TLC) | " | Complies |
| UV- spectroscopy | " | " |
| TEST OF PURITY | | |
| pH - value | 8.3-12.5 | 12.0 |
| Ethanol content in% (V / V) | 17-23 | 21 |
| Relative density d ²⁰₂₀ | 0.972-0.977 | 0.974 |
| Dry residue in% (m / m) | 0.06-0.4 | 0.13 |
| Microbiology in KBE/ml | GKZ<1 KBE/ml | Complies |
| Reaction with FeCl3 (HAB) | EEPH 26.01 | Orange color |
| Assessment: | In the points examined above the tested sample corresponds to the set requirements | |
| Comments | An additional test on alkaloids stearate 2002, test 2.3.1 with 10 ml evaporated to dryness Essence was negative, ie no alkaloids detected. | The ongoing stability tests and the analysis results according to findings Protocol S/0225 allow an extension of the expiration date and including 03.2010 |

| Product | **Propolis** | Test code: EPRO.08 |
|---|---|---|
| PROPERTIES | | |
| Appearance | EPRO.08 | Colorless |
| Odor | EPRO.08 | Typical wax-sweetish, slightly spicy |
| Taste | " | Typical wax-sweetish, slightly bitter, spicy |
| TEST OF IDENTITY | | |
| Thin-film chromatography (TLC) | " | Complies |
| UV- spectroscopy | " | Complies |
| TEST OF PURITY | | |
| pH - value | 3.5-11.5 | 8.00 |
| Ethanol content in% (V / V) | 16-30 | 20 |
| Relative density d ²⁰₂₀ | 0.968-0.982 | 0.975 |
| Dry residue in% (m / m) | Max 0.40 | 0.19 |
| Microbiology in KBE/ml | GKZ<1 | Complies |
| Methanol content in %(V/V) | ≤0.1 | 0.1 |
| 2-Propanol content in %(V/V) | ≤0.05 | <0.01 |
| | | |
| Assessment: | In the points examined above | |
| | the tested sample corresponds to the set requirements | |
| Comments | None | |

| Product | **Amigdala amara** | Test code: EAMY 26.06 |
|---|---|---|
| PROPERTIES | | |
| Appearance | EAMY 26.06 | Colorless |
| Odor | EAMY 26.06 | Typical of bitter almonds |
| Taste | " | Typical of bitter almonds, slightly spicy |
| TEST OF IDENTITY | | |
| Thin-film chromatography (TLC) | " | Complies |
| UV- spectroscopy | " | Complies |
| TEST OF PURITY | | |
| pH - value | 7.0-12.0 | 7.8 |
| Ethanol content in% (V / V) | 17-30 | 21 |
| Relative density d ²⁰₂₀ | 0.970-0.980 | 0.974 |
| Dry residue in% (m / m) | 0.03-0.6 | 0.10 |
| Microbiology in KBE/ml | GKZ<1 | Complies |
| Methanol content in %(V/V) | ≤0.1 | 0.1 |
| 2-Propanol content in %(V/V) | ≤0.05 | <0.01 |
| | | |
| Assessment: | In the points examined above the tested sample corresponds to the set requirements | |
| Comments | None | |

| Product | **Bryonia alba** | Test code: EBRY.05 |
|---|---|---|
| PROPERTIES | | |
| Appearance | EBRY.05 | Colorless |
| Odor | EBRY.05 | Sweetish, sour-fruity |
| Taste | " | Bitter, spicy |
| TEST OF IDENTITY | | |
| Thin-film chromatography (TLC) | " | Complies |
| UV- spectroscopy | " | Complies |
| TEST OF PURITY | | |
| pH - value | 7.0-12.0 | 8.00 |
| Ethanol content in% (V / V) | 16-30 | 21 |
| Relative density d ²⁰₂₀ | 0.970-0.980 | 0.975 |
| Dry residue in% (m / m) | 0.1-1.00 | 0.33 |
| Microbiology in KBE/ml | GKZ<1 | Complies |
| Assessment: | In the points examined above the tested sample corresponds to the set requirements | |
| Comments | None | |

### Processing methodology

Spagyric mother tincture of plants treated in accordance with dr. Zimpel method: Drosera, Catharanthus roseus, Mandragora officinalis, Hydrastis canadensis, Sambucus nigra, Tymus vulgaris, Ephedra, Propolis, Amigdala amara, Bryonia alba.

1-2 ml of all the spagyric mother tincture substances are mixed together (1 ml for the production of suppositories for small children under 36 months, 2 ml for the suppositories for children over 36 months). This process gives bottles with 10-20 ml of spagyric solution. The solution is mixed for about 3 minutes with a very weak blender paying attention not to create air bubbles and thus incorporate air. The solution is left to rest for about 2 minutes and if it begins to create deposits a further slight agitation in made (2-3 minutes). The speed of the mixture should be equal to that used for cell cultures (almost manual speed). Meanwhile, in a water bath is melt the solution of Witepsol H®15 (suppository mass) in these proportions: The concentration of the mixture as the active ingredient is supposed to be of 0.1 gr. (10 g: 100 g of total mass = x: volume of a 1 g suppository). Two kinds of suppositories are filled: for children under 36 months a suppository of maximum of 1g is used, while children over 36 months and for adults a 2g suppository is used.

The formation of suppositories for children under 36 months:
The proportion to fill approximately 98-100 individual units of suppository of 1 gr are: 10g of spagyric solution for every 90 grams of Witepsol H®15. The concentration of the spagyric solution towards the mass of suppositories must be equal to 10%. (The difference in filling the number of units of suppositories depends on the conditions and the contraction of the mixture between the mass and the spagyric solution). The concentration of the active principle in the mixture is 0.1 g per suppository. (10g: 100g of total mass = x : 1 g volume of one suppository).The recommended dosage vary based on weight and age of the children. The maximum dose that can be administered daily is 20 mg /kg. Never exceed the dose of 2 suppository per day for children under 36 months. Examples of administration: -0 Months to 2 months (minimum weight 5 kg to 8 kg): 1 suppository each day of 0.1 g (20 mg/kg): -4 months-6 months (weight 9 kg/12 kg): 2 suppository for a total of 0.2 grams per day (20 mg/kg) -8-12 Months up to 15 kg: 2 suppository for a total of 0.2 grams per day

Formation of suppositories for children over 36 months and adults:
The proportion are: 15g of solution every 80 grams of spagyric Witepsol H®15. This proportion can fill approximately 48-50 individual suppository units of 2 gr. The concentration of the spagyric solution towards the mass of the suppositories must be equal to 20%. The concentration the active principle in the mixture is 0.4 g for each suppository: (15 g: 100g of total mass = x: volume of a 2g suppository). Never exceed the administration dose of 2-3 suppository per day (20-40 mg / kg per day). Before dropping the spagiryc solution in the Witepsol H®15 a check has to be made to temperature that doesn't have to exceed 38°C. The suppository mass must melt gradually and have a look transparent, clear and uniform. After that the spagiryc solution has to be poured in the mass of the suppository unit until the solution ends. While the pouring is performed it can be noted that the solution of the suppositories mass tend to became torpid and then white, a little thick and opalescent. The mixture must be agitated for 30-45 seconds in a way that not too much air is incorporated. At this point is possible to fill the appropriate containers. Greater the time taken to fill, greater the chance to have the inappropriate contraction of the suppository (and that the last 2 suppository units are not complete). It is to avoid a rapid cooling of the suppository. It is preferable that the operation occurs at room temperature. To seal the suppositories is recommended but not mandatory, to wait after the solution began to solidify on the surface. The material should not be closed with material that contain metal (ex. Aluminium). The shelf life of the suppository is about 2 years. They have to be stored in a cool and dry place. The dosage is 1-3 suppositories a day, up to maximum 5 a day depending on weight and age (see above). Quality control checks to be performed are: -check for any leakage from the container of the suppositories, and that part of the head of the suppository and does not contain the presence of liquid. -checks required by the pharmacopoeia.

### Bibliography:

1. De Boer, A. G., Moolenaar, F., de Leede, J. & Breimer, D. D. (1982). Rectal drug administration: clinical pharmacokinetic considerations. Clinical Pharmacokinetics 7,285 - 311.[ISI][Medline]
2. Van Hoogdalem, E. J., De Boer, A. G. & Breimer, D. D. (1991). Pharmacokinetics of rectal drug administration. Part 1. General considerations and clinical applications of centrally acting drugs. Clinical Pharmacokinetics 21, 11-26.[ISI][Medline]
3. Moës, A. (1980). Biodisponibilité des formes d' administration rectale. Science and Technical Pharmaceutics 9, 263-88.
4. Henrich, M. (1980). Clinical topography of the proctodeum. Acta Anatomica 106, 161-70.[ISI][Medline]
5. Dellmann, H. D. & Brown, E. M. (1987). Textbook of Veterinary Histology, 3rd edn. Lea & Febiger, Philadelphia, PA.
6. Simanowski, U. A., Suter, P., Russel, R. M., Heller, M., Waldherr, R., Ward, R. et al. (1994). Enhancement of ethanol induced rectal mucosal hyper-regeneration with age in F344 rats. Gut 35, 1102-6.[Abstract/Free Full Text]
7. Kakemi, K., Arita, T. & Muranishi, S. (1965). Absorption and excretion of drugs. XXV. On the mechanism of rectal absorption of sulfonamides. Chemical and Pharmaceutical Bulletin 13, 861-9.
8. Bahia, M. F. (1991). Absorption of some cephalosporins by rectal route in rabbits. Bollettino Chimico Farmaceutico 130, 128-32.[Medline]
9. Nishimura, K. I.,Nozaki, Y., Yoshimi, A., Nakamura, S., Kitagawa, M., Kakeya, N. et al. (1985). Studies, on the promoting effects of carboxylic acid derivatives on the rectal absorption of ß-lactam antibiotics in rats. Chemical and Pharmaceutical Bulletin 33, 282-91.
10. Curry, S. H. (1977). Drug Disposition and Pharmacokinetics with a Consideration of Pharmacological and Clinical Relationship, 2nd edn. Blackwell Scientific, Oxford.
11. Matsumoto, Y., Watanabe, Y., Tojima, T., Murakoshi, R., Murakawi, C. & Matsumoto, M. (1989). Rectal absorption enhancement of gentamicin in rabbits from hollow type suppositories by sodium salicylate or sodium caprylate. Drug Design and Delivery 4, 247-56.[Medline]
12. Yata, N., Higashi, Y., Murakami, T., Yamajo, R., Wu, W.M., Taku, K. et al. (1983). A possible mechanism of absorption promoters. Journal of Pharmacobiodynamics 6,S78.
13. Nishihata, T., Rytting, J. H., Higuchi, T., Caldwell, L. J. and Selk, S. J. (1984). Enhancement of rectal absorption of water-soluble antibiotics in dogs. International Journal of Pharmaceutics 21,239-48.
14. Kakeya, N. (1985). Development of new adjuvants for enhanced rectal absorption. In Proceedings of the 14th International Congress of Chemotherapy, Kyoto, p. 2711.
15. Kawashima, S., Nishiura, N.,Noguchi, T. & Fujiwara, H. (1989). Studies on sustained-release suppositories. Effect of alginic acid addition on rectal absorption of bacampicillin in rabbits. Chemical and Pharmaceutical Bulletin 37, 766-70.
16. Nakanishi, K., Masukawa, T., Masada, M. & Nadai, T. (1994). Improvement of the rectal bioavailability of latamoxef sodium by adjuvants following administration of a suppository. Biological and Pharmaceutical Bulletin 17, 1496-500.
17. Van Hoogdalem, E. J., Geerts, J. A. M., De Boer, A. G. & Breimer, D. D. (1988). The influence of components on the rectal absorption of cefazolin in rats. Journal of Pharmacy and Pharmacology 40, 815-7.[ISI][Medline]
18. Van Hoogdalem, E. J., Wackwitz, A. T. E., De Boer, A. & Breimer, D. D. (1989). 3-Amino-1-hydroxypropylidene-1,1-diphosphonate (APD): a novel enhancer of rectal cefoxitin absorption in rats. Journal of Pharmacy and Pharmacology 41, 339-41.[ISI][Medline]
19. Unowsky, J., Behl, C. R., Beskid, G., Sattler, J., Halpern, J. & Cleeland, R. (1988). Effect of medium chain glycerides on enteral and rectal absorption of ß-lactam and aminoglycoside antibiotics.Chemotherapy 34, 272-6.[ISI][Medline]
20. Briedigkeit, W., Gü6res, E., Schneeweiss, B., Wachholz, E. & Wiegand, U. (1972). Zur Problematik einer rektalen Chloramphenikol-Applikation. Pädiatrie und Grenzgebiete 11, 241-51.
21. Farouk, A., Regdon, G., Mallalatif, G. & Abdel Hadi, I. A. (1984). Comparative study on ampicillin bioavailability from capsule and suppositories. Acta Pharmaceutica Hungarica 54,193-9.[Medline]
22. Sjü6vall, J., Westerlund, D., Alvan, G., Magni, L., Nord, C. E. & Sü6rstad, J. (1984). Rectal bioavailability of bacampicillin hydrochloride in man as determined by reversed-phase liquid chromatography. Chemotherapy 30, 137-47.[ISI][Medline]
23. Bergstrü6m, B. K. V., Bertilson, S. O. & Movin, G. (1988). Clinical evaluation of rectally administered ampicillin in acute otitis media.Journal of International Medical Research 16,376-85.
24. Davis, S. S., Burnham, W. R., Wilson, P. & O'Brien, J. (1985). Use of adjuvants for enhancement of rectal absorption of cefoxitin in humans. Antimicrobial Agents and Chemotherapy 28, 211-5.[Abstract/Free Full Text]
25. Van Hoogdalem, E. J., Wackwitz, A. T. E., De Boer, A. G., Cohen, A. F. & Breimer, D. D. (1989). Rate-controlled rectal absorption enhancement of cefoxitin by co-administration of sodium salicylate or sodium octanoate in healthy volunteers.British Journal of Clinical Pharmacology 27, 75-81.[ISI][Medline]
26. Pozzi, E., Luisetti, M. & Coppi, G. (1983). Sputum levels of erythromycin after rectal administration in adult patients with bronchitis. Current Therapeutic Research 33, 681-5.
27. De la Pierre, L., Acerbi, L., Gargantini, G., Manzoni, D. & Coppi, G. (1984). Livelli sierici di eritromicina dopo somministrazione rettale nella pratica pediatrica. Giornale Italiano di Chemioterapia 31, 229-32.[Medline]
28. Acerbi, L., De La Pierre, L., Perietti, L. & Coppi,G. (1983). Bioavailability studies of erythromycin administered by rectal route in paediatric patients. Chemoterapia 2, 200-2.
29. Stratchunsky, L. S., Nazarov, A. D., Firsov, A. A. & Petrachenkova, N. A. (1991). Age dependence of erythromycin rectal bioavailability in children. European Journal of Drug Metabolism and Pharmacokinetics Spec. No. 3, 321-3.
30. Luke, D. R., Foulds, G., Going, P. C., Melnik, G. & Lawrence, V. (1997). Rectal azithromycin in healthy subjects. In Expanding Indications of the New Macrolides, Azalides and Streptogramins (Zinner, S. H., Lowell, L. S., Acar, J. F. & Neu, H. D., Eds), pp. 474-7. Marcel Dekker, New York.
31. Bergan, T. & Arnold, E. (1980). Pharmacokinetics of metronidazole in healthy adult volunteers after tablets and suppositories. Chemotherapy 26, 231- 41.[ISI][Medline]
32. Mattila, J., Männisto, P. T., Mätyla, R., Nykänen, S. & Lamminsivu, U. (1983).Comparative pharmacokinetics of metronidazole and tinidazole as influenced by administration route. Antimicrobial Agents and Chemotherapy 23, 721- 5.[Abstract/Free Full Text]
33. Liedtke, R. and Haase, W. (1979). Steady-state pharmacokinetics of sulfamethoxazole and trimethoprim in man after rectal application. Arzneimittel-Forschung 29, 345-9.[Medline]
34. Abd Ej-Gawad, A. H., Ramadan, E. & Nouh, A. T. (1988). Formulation and evaluation of trimethoprim-sulfamethoxazole suppositories. Pharmaceutical Industry 50,257 -60.
35. Wagner, J. G., Carter, C. H. & Martens, I. J. (1968). Serum concentrations after rectal administration of lincomycin hydrochloride. Journal of Clinical Pharmacology and the Journal of New Drugs 8,154 -63.
36. Wagner, J. G., Leslie, L. G. & Gove, R. S. (1969). Relative absorption of both tetracycline and penicillin G administered rectally and orally in aqueous solution. Zeitschrift füCr Klinische Pharmakologie, Therapie und Toxikolgie 2, 44-51.
37. Pfaff, G., Zimmermann, T., Lach, P., Yeates, R., Simon, G. & Wildfeuer, A. (1993). Pharmacokinetics and tolerance of fluconazole suppositories in healthy volunteers. Arzneimittel-Forschung 43, 391-5.[Medline]
38. Joannides, L., Somogyi, A., Spicer, J., Heinzon, B., Tong, N., Franklin, C. et al. (1981). Rectal administration of metronidazole provides therapeutic plasma levels in postoperative patients. New England Journal of Medicine 305, 1569-70.[ISI][Medline]
39. E. Bergogne-Berezina,* and A. Bryskierb :" The suppository form of antibiotic administration: pharmacokinetics and clinical application"; The British Society for Antimicrobial Chemotherapy- Journal of Antimicrobial Chemotherapy (1999) 43, 177-185©, 1999
40. http://www.botanical.com/botanical/mgmh/b/brywhi77.html
41. http://www.botanical.com/botanical/mgmh/m/mandra10.html
42. http://findarticles.com/p/articles/mi_g2603/is_0002/ai_2603000234/pg_1?tag=artBody;col1
43. http://www.herbs2000.com/homeopathy/bryonia.htm
44. http://www.henriettesherbal.com/eclectic/kings/bryonia.html
45. http://www.naturalstandard.com/monographs/references/refs-bryonia.asp
46. http://aprendeenlinea.udea.edu.co/ova/?q=node/544
47. http://fichas.infojardin.com/perennes-anuales/catharanthus-roseus-catarantus-pervinca-dominica.htm
48. http://www.drugdigest.org/DD/DVH/HerbsWho/0,3923,552391 %7CEuropean+Mandrake,00. html
49. http://www.vithoulkas.com/content/view/104/9/lang,en/
50. http://www.pfaf.org/database/plants.php?Mandragora+officinarum
51. http://www.ibiblio.org/pfaf/cgi-bin/arr_html?Mandragora+officinarum#WEBREFS
52. http://ca.wikipedia.org/wiki/Pervinca
53. Sandro Pignatti, Flora d'Italia, Edagricole, Bologna 1982. ISBN 8850624492
54. T.G. Tutin, V.H. Heywood et Alii, Flora Europea, Cambridge University Press 1976. ISBN 052108489X
55. R. Dujany (2004), 291 p., Manuale pratico di omeopatia familiare e d'urgenza Red edizioni
56. D. Demarque, J. Jouanny, B. Poitevin, V. Saint Jean., Farmacologia e materia medica omeopatica Tecniche Nuove (1999), 480 p
57. George Britton ,The Biochemistry of Natural Pigments, By, Published by CUP Archive, 1983, ISBN 0521248922, 9780521248921, 366 p
58. Dewick Paul M., Chimica, biosintesi e bioattività delle sostanze naturali, Piccin-Nuova Libraria 2000, 500 p.
59. Capasso Francesco; Grandolini Giuliano; Izzo Angelo A. Fitoterapia. Impiego razionale delle droghe vegetali, Springer Verlag 2006, XXX-1032 p., brossura, 3 ed.
60. www.farmacovigilanza.org:
61. http://www.valtaro.it/erbeofficinali/timo.php
62. http://www.infoerbe.it/index.php?option=com_infoerbe&task=scheda&fld=BIBLIOGRAFIA&id e=209
63. http://it.wikipedia.org/wiki/Propoli
64. http://en.wikipedia.org/wiki/Rosy_Periwinkleouthern Herbals Limited
65. http://www.erbeofficinali.org/dati/q_scheda_res.php?nv_erba=MANDORLO
66. Sandro Pignatti, Flora d'Italia, Edagricole, Bologna 1982. ISBN 8850624492
67. T.G. Tutin, V.H. Heywood et Alii, Flora Europea, Cambridge University Press 1976. ISBN 052108489X
68. R. Dujany Manuale pratico di omeopatia familiare e d'urgenza Red edizioni
69. D. Demarque, J. Jouanny, B. Poitevin, V. Saint Jean Farmacologia e materia medica omeopatica Tecniche Nuove
70. Max tétau La materia medica omeopatica clinica e associazioni bioterapiche IPSA Editore
71. Paul M. Dewick"Chimica, biosintesi e bioattività delle sostanze naturali", Edizione Italiana, Piccin2001
72. F.Capasso, G. Grandolini, A.A.Izzo," Fitoterapia: impiego razionale delle droghe vegetali", Sprinter 2006
73. http///www.farmacovigilanza.org
74. http://www.valtaro.it/erbeofficinali/timo.php
75. www.infoerbe.it
76. http://www.infoerbe.it/index.php?option=com_infoerbe&task=scheda&fld=BIBLIOGRAFIA&id e=209
77. http://it.wikipedia.org/wiki/Propoli
78. http://en.wikipedia.org/wiki/Rosy_Periwinkleouthern Herbals Limited
79. http://www.erbeofficinali.org/dati/q_scheda_res.php?nv_erba=MANDORLO
80. http://www.infoerbe.it/index.php?option=com_infoerbe&task=scheda&fld=BIBLIOGRAFIA&id e=209
81. Marzio Pedretti, l'erborista moderno: manuale teorico-pratico di fitoterapia con spiegazione dell'effetto farmacologico delle piante medicinali, Studio Edizione,1998
82. The complete German Commission E Monographs, Therapeutic Guide to Herbal Medicines, American Botanical Council Austin, Texas e in coll. Integrative Medicine Communicatins Boston, Massachusetts, 1998, Pag. 316-317
83. http/www.pfaf.org/cgi-bin/pfaf/arr_html?Mandragora+officinarum (UK)
84. http://www.pfaf.org/database/plants.php?Mandragora+officinarum
85. http://www.ibiblio.org/pfaf/cgi-bin/arr_html?Mandragora+officinarum (US)

## Claims

1. Pharmaceutical forms of suppository type from 1 to 2 grams comprising of suppository mass and the following spagyric mother tincture: Drosera, Ephedra, Hydrastis canadensis, Thymus vulgaris, Amigdala amara, Propolis, Catharanthus roseus, Bryonia alba, Sambucus nigra, Mandragora officinalis.

2. Pharmaceutical suppository forms according to claim 1 in which the spagyric compounds are produced by utilizing the dr. Zimpel method in which the plant is produced using the homeopathic method.

3. Pharmaceutical forms as claims 1-3 comprising solid lipophilic semisynthetic excipients.

4. Pharmaceutical forms according to any of the previous claims in which the excipients used are dispersible in water, with and/or without the presence of non-ionic surfactants capable to form emulsions O/A.

5. Pharmaceutical forms according to any of previous claims comprising excipients selected form polyoxyethylene ,sorbitan moonostearate, sorbitan esters of fatty acids, polyoxyethylene esters of fatty acids, lipophilic of glycogelatine or glycostearate, semisynthetic excipients.

6. Pharmaceutical forms according to any of previous claims comprising saturated fatty acids, monounsaturated, di-glycerides and triglycerides and/or mixtures thereof.

7. Pharmaceutical forms according to any of previous claims comprising one or more of hydrogenated vegetable, fat, oil, cetyl alcohol and myristic as excipient in the formulation.

8. Pharmaceutical forms according to any of previous claims comprising mixtures of excipients that do not melt at the rectal temperature, but are dissected by dispersion or dissolution in the rectal mucus :e.g. polyethylene glycols.

9. Pharmaceutical forms according to any of previous claims comprising at least one of coconut oil, pam oil, nuts, olives, soya beans, seeds, wheat germ, sunflower, seed cotton, beef tallow or sardine oil.

10. Pharmaceutical forms according to any of previous claims produced by a method in which homeopathic dilutions corresponding to 1DH, 2DH are used.

11. Pharmaceutical forms according to any of previous claims comprising any combination of homeopathic diluitions and mixtures of spagyric plants according to the method of dr. Zimpel and/ or Krauss, that are suitable for phytotherapy, aryurvedic and other forms of alternative medicines and allopathic medicine.

12. Pharmaceutical forms according to any of previous claims in which Ephedra is Ephedra distachya, trifurcata Ephedra, Ephedra nevadensis, Ephedra American, Ephedra gerardiana Wall, nebrodensis Tineo Ephedra, Ephedra equisetina Bge.

13. Pharmaceutical forms according to any of previous claims comprising the aerial parts, flowers, leaves or roots of Ephedra.

## Patentansprüche

1. Pharmazeutische Formen des Typs 'Suppositorium' von 1 bis 2 Gramm bestehend aus den folgenden spagyrischen Muttertinkturen: Drosera, Ephedra, Hydrastis canadensis, Thymus vulgaris, Amigdala amara, Propolis, Catharanthus roseus, Bryonia alba, Sambucus nigra, Mandragora officinalis.

2. Pharmazeutische Formen des Typs 'Suppositorium' gemäß Anspruch 1,wobei die spagyrischen Zusammensetzungen homöopathischer Art sind, gemäss dem Dr. Zimpel-Verfahren.

3. Pharmazeutische Formen gemäß Ansprüchen 1 bis 3, umfassend lipophile halbsynthetische feste Hilfsstoffe.

4. Pharmazeutische Formen gemäß den vorangehenden Ansprüchen, bei welcher die verwendeten Hilfsstoffe in Wasser dispergierbar sind, mit oder ohne Anwesenheit von nicht-ionischen Tensiden mit der Fähigkeit, die Emulsionen 0 / W zu bilden.

5. Pharmazeutische Formen gemäß den vorangehenden Ansprüchen, **dadurch gekennzeichnet, dass** die Hilfsstoffe aus Polyoxyethylen, Sorbitanmonostearat, Sorbitanester von Fettsäuren, Polyoxyethylenestern von Fettsäuren bestehen können; es können demnach Hilfsstoffe wie beispielsweise lipophile Glycogelatin- oder Glykostearat zum Einsatz in Frage kommen.

6. Pharmazeutische Formen gemäß den vorangehenden Ansprüchen, enthaltend gesättigte Fettsäuren, einfach ungesättigte Fettsäuren, Di-Glyceride und Triglyceride und / oder deren Mischungen.

7. Pharmazeutische Formen gemäß den vorangehenden Ansprüchen, enthaltend ein oder mehrere hydrierte Vegetabilien, Fett, Öl, Cetylalkohol und Myristin als Hilfsstoff in der Formulierung

8. Pharmazeutische Formen gemäß den vorangehenden Ansprüchen, enthaltend Mischungen von Hilfsstoffen, welche bei der Rektaltemperatur nicht schmelzen, sondern durch Dispergieren oder Auflösen im Rektumschleim zerlegt werden, z.B. Polyethylenglykolen.

9. Pharmazeutische Formen gemäß den vorangehenden Ansprüchen, enthaltend mindestens eines der ff. Substanzen: Kokosnussöl, Palmöl, Nüssen, Oliven, Sojabohnen, Samen, Weizenkeimen, Sonnenblumen, Baumwollsamen, Rindertalg oder Sardinenöl.

10. Pharmazeutische Formen gemäß den vorangehenden Ansprüchen, hergestellt durch ein Verfahren, bei dem homöopathische Verdünnungen entsprechend 1DH, 2DH verwendet werden.

11. Pharmazeutische Formen gemäß den vorangehenden Ansprüchen, enthaltend beliebige Kombinationen von homöopathischen Verdünnungen und Gemischen von spagyrischen Pflanzen nach dem Verfahren von Dr. Zimpel und / oder Krauss, welche für aryurvedische und andere Formen der alternativen Medizin und der allopathischen Medizin geeignet sind.

12. Pharmazeutische Formen gemäß den vorhergehenden Ansprüchen, wobei unter Ephedra ff. Formen verstanden werden: Ephedra distachya, trifurcata Ephedra, Ephedra nevadensis, Ephedra American, Ephedra gerardiana Wall, nebrodensis Tineo Ephedra, Ephedra equisetina Bge.

13. Pharmazeutische Formen gemäß den vorangehenden Ansprüchen, enthaltend oberirdische Pflanzenteile, Blüten, Blätter oder Wurzeln von Ephedra

## Revendications

1. Formule pharmaceutique en suppositoires de 1 à 2 grammes comprenant de la masse suppositoire et des suivantes teintures d'origine spagyric : Drosera, Ephedra, Hydrastis canadensis, Thymus vulgaris, Amigdala amara, Propolis, Catharanhus roseus, Bryonia alba, Sambucus nigra, Mandragora officinalis.

2. Suppositoires pharmaceutiques, selon la revendication et en conformité du point n. 1, dont les composés de spagyric sont produits selon la méthode du docteur Zimpel où toute plante est produite selon la méthode homéopathique.

3. Formule pharmaceutique, selon la revendication et en conformité des points n. 1 et 2, qui contient des excipients semi-synthétiques lipophiles fermes.

4. Formule pharmaceutique, selon et en conformité de toute revendication précédente, dans laquelle les excipients employés peuvent être dispersés dans l'eau, si bien en présence que non de non-ioniques surfactants capables d'engendrer des émulsions O/A.

5. Formule pharmaceutique selon et en conformité de toute revendication précédente y compris les excipients de formules sélectionnées de polyoxyethylene, sorbitan monostearate, sorbitan esters des acides gras, popyoxyethylene esters des acides gras, excipients lipophiles de glycogelatine ou glycostearate.

6. Formule pharmaceutique, selon et en conformité de toute revendication précédente, qui a aussi d'acides gras saturés, mono insaturés, d-glycerides et triglycérides ou leur mélange.

7. Formule pharmaceutique, selon et en conformité de toute revendication précédente, qui comprend un ou plusieurs éléments tels que légumes hydrogénés, gras, huile, alcool-cetyl et myristic comme excipients dans sa formulation.

8. Formule pharmaceutique, selon et en conformité de toute revendication précédente, comprenant des mélanges de excipients qui ne fondent pas à la température du rectum, néanmoins sont dispersés ou dissous dans la muqueuse du rectum : polyethilène glycols.

9. Formule pharmaceutique, selon et en conformité de toute revendication précédente, qui comprend au moins un des huiles suivants : coco, palmier, noix, olives, soya, graines oléagineuses, germes de blé, tournesol, graines de coton, suif de boeuf, sardine.

10. Formule pharmaceutique, selon et en conformité de toute revendication précédente, produite par une méthode de dilutions homéopathiques conformes à IDH, 2DH.

11. Formule pharmaceutique selon et en conformité de toute revendication précédente y compris toutes combinaisons de dilutions homéopathiques et mélanges de plantes spagyric selon les méthodes du docteur Zimpel ou/et du docteur Krauss, qui sont convenables pour aryurvedic ou d' autres produits de médecine alternative ou de médecine allopathic.

12. Formule pharmaceutique, selon et en conformité de toute revendication précédente, dans laquelle Ephedra est de l'Ephedra distachya, de trifurcata Ephedra, de l'Ephedra nevadentis, de l' Ephedra American, de l'Ephedra gerardiana Wall, de nebrodensis Tineo Ephedra, de l'Ephedra equisetina Bge..

13. Formule pharmaceutique selon et en conformité de toute revendication précédente comprenant les parties éthérées, les fleurs, les feuilles ou les racines d' Ephedra.
